# FASCICULE DE BREVET EUROPEEN

(11) **EP 1 042 491 B1**
(45) Date de publication et mention de la délivrance du brevet: **03.05.2006**
(21) Numéro de dépôt: 98963587.5
(22) Date de dépôt: 22.12.1998
(51) Int. Cl.: C12N 15/82, C12N 15/29, C07K 14/415, C07K 14/21, C12N 15/62, C12Q 1/68, A01H 5/00

(54) **PROMOTEUR H3C4 DE MAIS ASSOCIE AU PREMIER INTRON DE L'ACTINE DE RIZ, GENE CHIMERE LE COMPRENANT ET PLANTE TRANSFORMEE**
PROMOTOR DES H3C4 GENS AUS MAIS, VERBUNDEN MIT ERSTEM ACTIN-INTRON AUS REIS; CHIMÄRES GEN WELCHES DIESES BEINHALTET UND TRANSFORMIERTE PFLANZEN
MAIZE H3C4 PROMOTER ASSOCIATED WITH FIRST RICE ACTIN INTRON, CHIMERIC GENE CONTAINING IT AND TRANSFORMED PLANT

(30) Priorité: 24.12.1997 FR 9716726
(43) Date de publication de la demande: 11.10.2000
(73) Titulaire: Bayer CropScience S.A., 69009 Lyon (FR)
(72) Inventeur: DEROSE, Richard, F-91000 Evry (FR); FREYSSINET, Georges, F-69450 St. Cyr Au Mont d'Or (FR)
(74) Mandataire: Nowak, Alexander
(86) Numéro de dépôt international: PCT/FR1998/002820
(87) Numéro de publication internationale: WO 1999/034005

(56) Documents cités:
- EP-A- 0 508 909
- EP-A- 0 652 286
- WO-A-91/09948
- WO-A-92/04449
- WO-A-97/04103
- BRIGNON, P., ET AL.: "nuclease sensitivity and functional analysis of a maize histone H3 gene promoter" PLANT MOLECULAR BIOLOGY, vol. 22, 1993, pages 1007-1015, XP002078982 cité dans la demande
- CHAUBET, N., ET AL.: "organization of the histone H3 and H4 multigenic families in maize and in related genomes" MOLECULAR AND GENERAL GENETICS, vol. 219, 1989, pages 404-412, XP002078983
- LEPETIT M ET AL: "REPLICATION-INDEPENDENT CIS-ACTING ELEMENT OF A MAIZE HISTONE GENE PROMOTER" PLANT SCIENCE, vol. 89, 1993, pages 177-184, XP000672028
- TERADA R ET AL: "A WHEAT HISTONE H3 PROMOTER CONFERS CELL DEVISION-DEPENDENT AND INDEPENDENT EXPRESSION OF THE GUS A GENE IN TRANSGENIC RICE PLANTS" PLANT JOURNAL, vol. 3, no. 2, 1993, pages 241-252, XP002030453

## Description

La présente invention concerne une nouvelle séquence de régulation en 5' permettant l'expression dans les plantes monocotylédones d'une séquence hétérologue à ladite séquence de régulation codant pour une protéine d'intérêt. La présente invention concerne également un gène chimère comprenant ladite séquence de régulation, une séquence hétérologue codant pour une protéine d'intérêt et une séquence de régulation en 3' permettant l'expression de la protéine d'intérêt dans une cellule végétale de plante monocotylédone, ainsi qu'une plante monocotylédone transformée comprenant ledit gène chimère et les moyens nécessaires à la transformation des cellules végétales et des plantes.

Différents promoteurs permettant l'expression de séquences codant pour des protéines d'intérêt dans les plantes sont connus, décrits dans la littérature, et ont déjà permis le développement à un stade commercial de plantes modifiées par génie génétique. Il s'agit de séquences promotrices de gènes s'exprimant naturellement dans les plantes en particulier des promoteurs d'origine bactérienne, virale ou végétale tels que, par exemple, celui d'un gène de la petite sous unité de ribulose-bisphosphate carboxylase/oxygénase (US 4,962,028) ou d'un gène de virus de plante tel que, par exemple, celui de la mosaïque du choux fleur (US 5,352,605). Des promoteurs permettant l'expression de gènes hétérologues dans les plantes sont notamment décrits dans les brevets et demandes de brevet suivants: US 5,086,169, EP 0 353 908 US 5,139,954, US 5,378,619, US 5,563,328, US 5,589,583, US 5,633,363, US 5,633,439, US 5,633,440, US 5,633,447, US 3,635,618, US 5,639,948 et US 5,639,952.

Toutefois, certains de ces promoteurs, et plus particulièrement les promoteurs d'origine végétale ne sont pas fonctionnels dans les monocotylédones.

On connaît par exemple des promoteurs d'histone *d'Arabidopsis sp.* décrits dans la demande de brevet EP 0 507 698, particulièrement efficaces pour permettre l'expression d'un gène hétérologue dans des plantes dicotylédones comme le tabac, le colza ou le soja, qui ne sont pas fonctionnels dans les monocotylédones comme le maïs.

Le promoteur de l'actine de riz est un promoteur connu pour permettre l'expression de gènes hétérologues dans les plantes monocotylédones (US 5,641,876). Toutefois, le problème subsiste d'identifier de nouvelles séquences de régulation en 5' fonctionnelles pour l'expression de séquences hétérologues dans les plantes monocotylédones.

La présente invention concerne une nouvelle séquence d'ADN, élément de régulation en 5' permettant l'expression d'un gène hétérologue dans une cellule végétale de plante monocotylédone, la dite séquence d'ADN comprenant dans le sens de la transcription une première séquence d'ADN, fragment fonctionnel de la séquence du promoteur H3C4 de maïs, et une deuxième séquence d'ADN, fragment fonctionnel de la séquence du premier intron de l'actine de riz.

La séquence du promoteur H3C4 de maïs est notamment décrite par Brignon & coll. (Plant. Mol. Biol., 22: 1007-1015, 1993). Il s'agit du fragment *AluI* du promoteur H3C4 de maïs, d'environ 1 kb, correspondant aux bases -7 à -1029 relatives à l'ATG de la séquence codant pour l'histone H3C4 de maïs.

La séquence du premier intron de l'actine de riz est notamment décrite dans le brevet US 5,641,876.

Par fragment fonctionnel, on entend selon l'invention toute séquence d'ADN issue de la séquence du promoteur H3C4 de maïs ou de la séquence du premier intron de l'actine de riz, reproduisant la fonction de la séquence d'où elle est issue.

Selon un mode de réalisation de l'invention, le fragment fonctionnel de la séquence du promoteur H3C4 de maïs comprend la séquence d'ADN décrite par l'identificateur de séquence n° 1 (SEQ ID NO:1) ou une séquence homologue de ladite séquence. De manière préférentielle, le fragment fonctionnel de la séquence du promoteur H3C4 de maïs consiste en la séquence d'ADN décrite par l'identificateur de séquence n° 1.

Selon un mode de réalisation de l'invention, le fragment fonctionnel du premier intron de l'actine de riz comprend la séquence d'ADN décrite par l'identificateur de séquence n° 2 (SEQ ID NO:2) ou une séquence homologue de ladite séquence. De manière préférentielle, le fragment fonctionnel du premier intron de l'actine de riz consiste en la séquence d'ADN décrite par l'identificateur de séquence n° 2.

La séquence d'ADN, élément de régulation en 5' selon l'invention peut comprendre en outre entre la première et la deuxième séquence d'ADN, des fragments d'ADN neutres, généralement nécessaires à la construction de la séquence selon l'invention. Il s'agit de fragments d'ADN comprenant jusqu'à 30 paires de bases, préférentiellement jusqu'à 20 paires de bases. Par fragments d'ADN neutres, on entend selon l'invention des fragments d'ADN qui ne viennent pas modifier de manière substantielle les fonctions respectives des première et deuxième séquences d'ADN de la séquence selon l'invention.

Selon un mode préférentiel de réalisation de l'invention, la séquence d'ADN selon l'invention comprend la séquence d'ADN représentée par l'identificateur de séquence n° 3 (SEQ ID NO:3) ou une séquence homologue de ladite séquence. Plus préférentiellement, la séquence selon l'invention consiste en la séquence d'ADN représentée par l'identificateur de séquence n° 3.

Par " homologue ", on entend selon l'invention une séquence d'ADN présentant une ou plusieurs modifications de séquence par rapport à la séquence d'ADN de référence décrite par les identificateurs de séquence n° 1, 2 ou 3, et reproduisant la fonction des séquences précitées. Ces modifications peuvent être obtenues selon les techniques usuelles de mutation, ou encore en choisissant les oligonucléotides synthétiques pouvant être employés dans la préparation de ladite séquence par hybridation. De manière avantageuse, le degré d'homologie sera d'au moins 70 % par rapport à la séquence de référence, de préférence d'au moins 80 %, plus préférentiellement d'au moins 90 %.

La présente invention concerne également un gène chimère (ou cassette d'expression) comprenant une séquence codante ainsi que des éléments de régulation en position 5' et 3' hétérologues pouvant fonctionner dans les cellules végétales de plantes monocotylédones, dans laquelle les éléments de régulation en 5' comprennent la séquence d'ADN selon l'invention définie ci-dessus.

Par "cellule végétale", on entend selon l'invention toute cellule issue d'une plante monocotylédone et pouvant constituer des tissus indifférenciés tels que des cals, des tissus différenciés tels que des embryons, des parties de plantes monocotylédones, des plantes monocotylédones ou des semences.

On entend par "plante monocotylédone" selon l'invention, tout organisme multicellulaire différencié capable de photosynthèse et dont la graine a un embryon pourvu d'un seul cotylédon, plus particulièrement des plantes de culture destinées ou non à l'alimentation animale ou humaine, comme par exemple le blé, l'orge, l'avoine, le riz, le maïs, le sorgho, la canne à sucre, etc.

Selon l'invention, on peut également utiliser, en association avec la séquence de régulation promotrice selon l'invention, d'autres séquences de régulation, qui sont situées entre le promoteur et la séquence codante, telles les séquences codant pour des peptides de transit, soit simples, soit doubles, et dans ce cas éventuellement séparés par une séquence intermédiaire, c'est à dire comprenant, dans le sens de la transcription, une séquence codant pour un peptide de transit d'un gène végétal codant pour une enzyme à localisation plastidiale, une partie de séquence de la partie mature N-terminale d'un gène végétal codant pour une enzyme à localisation plastidiale, puis une séquence codant pour un second peptide de transit d'un gène végétal codant pour une enzyme à localisation plastidiale constituée d'une partie de séquence de la partie mature N-terminale d'un gène végétal codant pour une enzyme à localisation plastidiale, tels que décrit dans la demande EP 0 508 909. Comme peptide de transit, on peut également citer le peptide signal du gène PR-1a du tabac décrit par Cornelissen & coll.

Comme séquence de régulation terminatrice ou de polyadénylation, on peut utiliser toute séquence correspondante d'origine bactérienne, comme par exemple le terminateur nos *d'Agrobacterium tumefaciens,* ou encore d'origine végétale, comme par exemple un terminateur d'histone tel que décrit dans la demande EP 0 633 317.

La séquence codante du gène chimère selon l'invention peut comprendre toute séquence codant pour une protéine d'intérêt que l'on souhaite exprimer dans une cellule végétale ou une plante monocotylédone.

Il peut s'agir d'un gène codant pour un marqueur de sélection comme d'un gène conférant à la plante monocotylédone transformée de nouvelles propriétés agronomiques, ou d'un gène d'amélioration de la qualité agronomique de la plante monocotylédone transformée.

Parmi les gènes codant pour des marqueurs de sélection, on peut citer les gènes de résistance aux antibiotiques, les gènes de tolérance aux herbicides (bialaphos, glyphosate ou isoxazoles), des gènes codant pour des enzymes facilement identifiables comme l'enzyme GUS, des gènes codant pour des pigments ou des enzymes régulant la production de pigments dans les cellules transformées. De tels gènes marqueurs de sélection sont notamment décrits dans les demandes de brevet WO 91/02071 et WO 95/06128.

Parmi les gènes conférant de nouvelles propriétés agronomiques aux plantes monocotylédones transformées, on peut citer les gènes conférant une tolérance à certains herbicides, ceux conférant une tolérance à certains insectes, ceux conférant une tolérance à certaines maladies, etc. De tels gènes sont notamment décrits dans les demandes de brevet WO 91/02071 et WO 95/06128.

Comme séquence de régulation terminatrice ou de polyadénylation, on peut utiliser toute séquence correspondante d'origine bactérienne, comme par exemple le terminateur nos d*'Agrobacterium tumefaciens,* ou encore d'origine végétale, comme par exemple un terminateur d'histone tel que décrit dans la demande EP 0 633 317.

La présente invention est particulièrement appropriée pour l'expression de gènes conférant une tolérance à certains herbicides aux cellules végétales et aux plantes monocotylédones transformées. Parmi les gènes conférant une tolérance à certains herbicides, on peut citer le gène *Bar* conférant une tolérance au bialaphos, le gène codant pour une 5-enolpyruvylshikimate-3-phosphate synthase (EPSPS) appropriée conférant une résistance aux herbicides ayant l'EPSPS comme cible comme le glyphosate et ses sels (US 4,535,060, US 4,769,061, US 5,094,945, US 4,940,835, US 5,188,642, US 4,971,908, US 5,145,783, US 5,310,667, US 5,312,910, US 5,627,061, US 5,633,435, FR 2 736 926), le gène codant pour la glyphosate oxydoréductase (US 5,463,175), ou encore un gène codant pour une Hydroxy-phenyl pyruvate dioxygenase (HPPD) conférant une tolérance aux herbicides ayant pour cible l'HPPD comme les isoxazoles, notamment l'isoxafutole (FR 95 06800, FR 95 13570), les dicétonitriles (EP 496 630, EP 496 631) ou les tricétones, notamment la sulcotrione (EP 625 505, EP 625 508, US 5,506,195). De tels gènes codant pour une HPPD conférant une tolérance aux herbicides ayant pour cible l'HPPD sont décrits dans la demande de brevet WO 96/38567 et dans la demande de brevet non publiée FR 97 14264, déposée le 7 novembre 1997 novembre 1997.

Parmi les gènes codant pour une EPSPS appropriée conférant une résistance aux herbicides ayant l'EPSPS comme cible, on citera plus particulièrement le gène codant pour une EPSPS végétale, en particulier de maïs, présentant deux mutations 102 et 106, décrit dans la demande de brevet FR 2 736 926, dénommé ci-dessous EPSPS double mutant, ou encore le gène codant pour une EPSPS isolée d*'Agrobacterium* décrit par les séquences ID 2 et ID 3 du brevet US 5,633,435, dénommé ci-dessous CP4.

Parmi les gènes codant pour une HPPD conférant une tolérance aux herbicides ayant pour cible l'HPPD, on citera plus particulièrement l'HPPD de *Pseudomonas* et celle d*'Arabidopsis,* décrites dans la demande de brevet WO 96/38567.

Dans les cas des gènes codant pour EPSPS ou HPPD, et plus particulièrement pour les gènes ci-dessus, la séquence codant pour ces enzymes est avantageusement précédée par une séquence codant pour un peptide de transit, en particulier pour le peptide de transit dit peptide de transit optimisé décrit dans les brevets US 5,510,471 ou US 5,633,448.

Selon un mode préférentiel de réalisation de l'invention, le gène chimère selon l'invention comprend dans le sens de la transcription, une séquence de régulation en 5' selon l'invention telle que définie ci-dessus, liée de manière fonctionnelle à une séquence codant pour une protéine de fusion peptide de transit/protéine d'intérêt, lié de manière fonctionnelle à une séquence de régulation en 3', les différents éléments du gène chimère étant définis ci-dessus, la protéine d'intérêt étant de préférence une enzyme conférant une tolérance à certains herbicide, plus préférentiellement les enzymes de type EPSPS ou HPPD définis ci-dessus.

Des séquences codant pour des protéines de fusion peptide de transit/EPSPS, et plus particulièrement OTP/EPSPS double mutant sont notamment décrites dans les brevets US 4,940,835, US 5,633,448 et FR 2 736 926.

Pour la protéine de fusion OTP/CP4, l'homme du métier saura construire le gène correspondant en prenant la séquence codant pour le CP4 décrite dans le brevet US 5,633,435 et en suivant le mode opératoire décrit dans les brevets US 4,940,835, US 5,633,448 et FR 2 736 926 ou dans les exemples ci-dessous. La présente invention concerne également un gène chimère comprenant dans le sens de la transcription, une séquence de régulation en 5' appropriée pour assurer l'expression d'un gène hétérologue dans une cellule végétale, liée de manière fonctionnelle à une séquence codant pour une protéine de fusion OTP/CP4, liée de manière fonctionnelle à une séquence de régulation en 3'. Les éléments de régulation en 5' comprennent non seulement les éléments de régulation en 5' selon l'invention défini ci-dessus, mais également tous les éléments de régulation appropriés pour permettre l'expression de gènes hétérologues dans les cellules végétales de plantes monocotylédones connus de l'homme du métier ou à venir, et notamment ceux décrits plus haut.

Les séquences codant pour des protéines de fusion peptide de transit/HPPD sont décrites dans la demande de brevet WO 96/38567.

La présente invention concerne également un vecteur de clonage ou d'expression pour la transformation d'une cellule végétale ou d'une plante monocotylédone, les cellules végétales et plantes transformées contenant au moins un gène chimère tel que défini ci-dessus. Le vecteur selon l'invention comprend outre le gène chimère ci-dessus, au moins une origine de réplication. Ce vecteur peut être constitué par un plasmide, un cosmide, un bactériophage ou un virus, transformés par l'introduction du gène chimère selon l'invention. De tels vecteurs de transformation de cellules végétales et de plantes monocotylédones sont bien connus de l'homme du métier et largement décrits dans la littérature. De manière préférentielle, le vecteur de transformation des cellules végétales ou des plantes selon l'invention est un plasmide.

L'invention a encore pour objet un procédé de transformation des cellules végétales par intégration d'au moins un fragment d'acide nucléique ou un gène chimère tels que définis ci-dessus, transformation qui peut être obtenue par tout moyen connu approprié avec le vecteur selon l'invention.

Une série de méthodes consiste à bombarder des cellules ou des tissus cellulaires avec des particules auxquelles sont accrochées les séquences d'ADN. Une autre série de méthodes consiste à utiliser comme moyen de transfert dans la plante un gène chimère inséré dans un plasmide Ti d*'Agrobacterium tumefaciens* ou Ri d*'Agrobacterium rhizogenes.* D'autres méthodes peuvent être utilisées telles que la micro-injection ou l'électroporation, ou encore la précipitation directe au moyen de PEG.

L'homme du métier fera le choix de la méthode appropriée en fonction de la nature de la cellule végétale ou de la plante.

La présente invention a encore pour objet les cellules végétales ou plantes, transformées et contenant au moins un gène chimère selon l'invention défini ci-dessus.

La présente invention a encore pour objet les plantes contenant des cellules transformées, en particulier les plantes régénérées à partir des cellules transformées. La régénération est obtenue par tout procédé approprié qui dépende de la nature de l'espèce.

Pour les procédés de transformation des cellules végétales et de régénération des plantes monocotylédones, on citera notamment Gordon-Kamm, W.J. et al. (*Transformation of Maize Cells and Regeneration of Fertile Transgenic Plants,* The Plant Cell, vol. 2, 603-618, July 1990), et les brevets et demandes de brevet suivants: US 5,177,010, US 5,187,073, EP 267,159, EP 604 662, EP 672 752, US 4,945,050, US 5,036,006, US 5,100,792, US 5,371,014, US 5,478,744, US 5,484,956, US 5,508,468, US 5,538,877, US 5,554,798, US 5,489,520, US 5,510,318, US 5,204,253, US 5,405,765, EP 442 174, EP 486 233, EP 486 234, EP 539 563, EP 674 725, WO 91/02071 et WO 95/06128.

La présente invention concerne également les plantes transformées issues de la culture et/ou du croisement des plantes régénérées ci-dessus, ainsi que les graines de plantes transformées.

Dans le cas où le gène chimère selon l'invention comprend une séquence codant pour une enzyme conférant une tolérance à un herbicide particulier, la présente invention concerne également un procédé de contrôle des mauvaises herbes dans une surface d'un champ comprenant des graines ou des plantes transformées avec le dit gène chimère selon l'invention, lequel procédé consiste à appliquer dans la dite surface du champ une dose dudit herbicide particulier toxique pour les dites mauvaises herbes, sans toutefois affecter de manière substantielle les graines ou plantes transformée avec le dit gène chimère selon l'invention comprenant la dite séquence codant pour une enzyme conférant une tolérance au dit herbicide particulier.

La présente invention concerne également un procédé de culture des plantes transformées selon l'invention avec un gène chimère selon l'invention comprenant une séquence codant pour une enzyme conférant une tolérance à un herbicide particulier défini ci-dessus, lequel procédé consiste à planter les graines des dites plantes transformées dans une surface d'un champ approprié pour la culture des dites plantes, à appliquer sur la dite surface du dit champ une dose toxique pour les mauvaises herbes du dit herbicide particulier en cas de présence de mauvaises herbes, sans affecter de manière substantielle les dites graines ou les dites plantes transformées, puis à récolter les plantes cultivées lorsqu'elles arrivent à la maturité souhaitée et éventuellement à séparer les graines des plantes récoltées.

Dans les deux procédés ci-dessus, l'application de l'herbicide particulier peut être faite selon l'invention, tant en présemis, en prélevée qu'en postlevée de la culture.

De manière avantageuse, l'enzyme de tolérance à un herbicide est une EPSPS appropriée, et dans ce cas l'herbicide est le glyphosate ou ses sels, ou l'enzyme est une HPPD et l'herbicide est choisi parmi les isoxazoles, notamment l'isoxafutole, les dicétonitriles ou les tricétones, notamment la sulcotrione Les exemples ci-après permettent d'illustrer l'invention sans chercher à en limiter la portée.

### 1. Construction d'un gène chimère avec une séquence codant pour une HPPD:

Les plasmides ci-dessous sont préparer de manière à créer une cassette d'expression comprenant un promoteur histone H3C4 de maïs associé avec la région 5' non traduite du premier intron du gène d'actine de riz *(ActI)* décrit par Mc Elroy D. *et al.* (Plant Molecular Biology 15: 257-268 (1990)) conduisant l'expression du gène OTP-HPPD de *Pseudomonas fluorescens.*

### pRPA-RD-195

Le plasmide pRPA-RD-195 est un dérivé du plasmide pUC-19 qui contient un site de clonage multiple modifié. Les oligonucléotides complémentaires 1 et 2 ci-dessous sont hybridés à 65°C pendant 5 minutes, suivi d'un refroidissement lent jusqu'à 30°C pendant 30 minutes.:

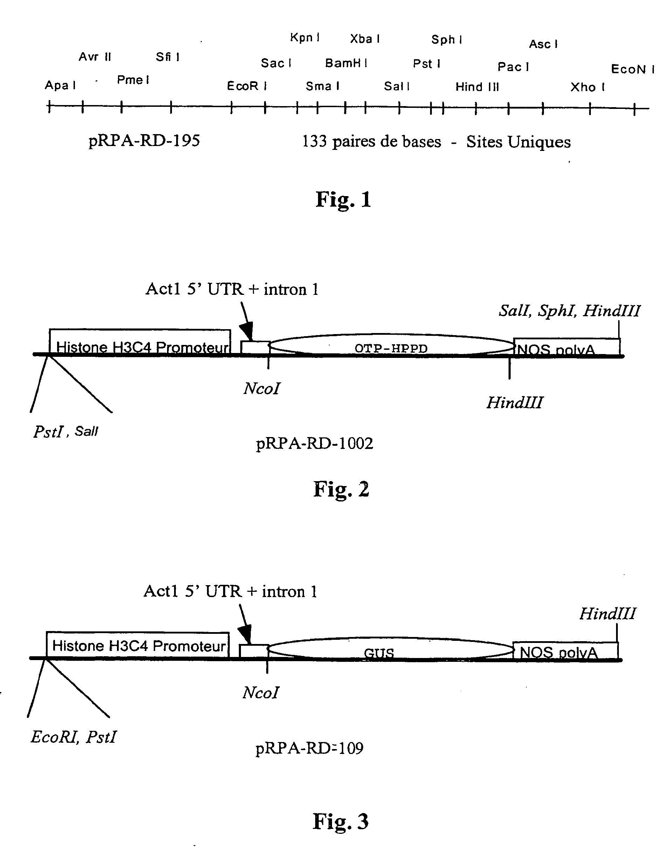

Les oligonucléotides hybridés sont rendus double brins en employant le fragment klenow de la DNA polymérase 1 de *E.coli* pour étendre les extrémités 3' de chaque oligo en employant les conditions standard recommandées par le fabriquant (New England Biolabs). L'oligo double brins obtenu est ensuite lié dans le plasmide pUC-19 préalablement digéré avec les enzymes de restriction *EcoRI* et *HindIII* et rendus bords franc en employant le fragment klenow de la DNA polymérase 1 de *E.coli.* On obtient ainsi un vecteur de clonage comprenant un site de clonage multiple de manière à faciliter l'introduction de cassettes d'expression dans un vecteur plasmide d*'Agrobacterium tumefaciens* (figure 1).

### pRPA-RD-2010:

Insertion de la séquence "promoteur H4A748-OTP-gène d'EPSPS double mutant" de pRPA-RD-173 dans le plasmide pRPA-RD-195.

Le plasmide pRPA-RD-195 est digéré avec l'enzyme de restriction *SacI* et déphosphorylé avec de la phosphatase alcaline des intestins de veau (New England Biolabs). Le plasmide pRPA-RD-173 (décrit dans le brevet FR 2 736 926) est digéré avec l'enzyme de restriction *SacI* et le fragment d'ADN contenant le gène d'EPSPS est purifié et lié dans le plasmide pRPA-RD-195 préparé précédemment. Le colone obtenu contient plusieurs sites de restriction uniques encadrant le gène d'EPSPS double mutant.

### pRPA-RD-1002

Création d'une cassette d'expression OTP-HPPD pour usage dans les monocotylédones. Le plasmide pRP-P contient le peptide de transit optimisé (OTP) lié à l'HPPD de *Pseudomonas fluorescens* suivi par le site de polyadénylation de la nopaline synthase tel que décrit dans la demande de brevet WO 96/38567. Les éléments du plasmide pRP-P sont les suivants:
- le peptide de transit optimisé (OTP) décrit dans les brevets US 5,510,471 et US 5,633,448; cet OTP étant constitué des 171 pb du peptide de transit de la petite sous unité de la Ribulose 1,5 bisphosphate carboxylase /oxygénase d*'Helianthus annuus* (Waksman G. *et al* 1987. Nucleics acids Res. 15: 7181) suivies des 66 pb de la partie mature de la petite sous unité de la Ribulose 1,5 bisphosphate carboxylase /oxygénase de *Zea mays* (Lebrun et *al* 1987. Nucleics acids Res. 15: 4360) elles mêmes suivies des 150 pb du peptide de transit de la petite sous unité de la Ribulose 1,5 bisphosphate carboxylase/ oxygénase de *Zea mays* (Lebrun *et al* 1987. Nucleics acids Res. 15: 4360); L'ensemble fait donc 387 pb;
- la région codante de l'HPPD de *Pseudomonas fluorescens* décrite dans la demande de brevet WO 96/38567; et
- le terminateur du gène de la nopaline synthase (nos) (zone de polyadenylation du gène *nos* isolé de pTi 37, 250 pb ; Bevan M. et al. Nucleics Acids Res. 11: 369-385).

Le plasmide pRP-P est digéré avec l'enzyme de restriction *BstEII,* traité avec le fragment klenow de la DNA polymerase I de *E. coli* pour rendre le fragment à bords francs, puis digéré avec l'enzyme de restriction *NcoI.* Le fragment d'ADN obtenu contenant la région codante OTP-HPPD d'environ 1,5 kb est ensuite purifié. Le plasmide pRPA-RD-2010 obtenu précédemment est digéré avec l'enzyme de restriction *BlpI,* traité avec le fragment klenow de la DNA polymérase I de *E. coli* pour obtenir un fragment à bords francs, puis digéré avec l'enzyme de restriction *NcoI.* Le fragment d'ADN obtenu comprenant les séquences du vecteur plasmide, le promoteur H3C4 associé avec la région 5'non traduite et le premier intron du gène d'actine de riz est purifié et le site de polyadénylation NOS est purifié. Les deux fragments d'ADN purifiés sont liés de manière à créer une cassette d'expression OTP-HPPD comprenant le promoteur d'histone H3C4 de maïs (Brignon & coll) associé avec la région non traduite en 5' et le premier intron du gène d'actine de riz *(ActI)* (Act1 5' UTR + intron 1) pour contrôler l'expression de la région codante OTP-HPPD incorporant le site de polyadénylation NOS (NOS polyA) (figure 2).

### 2. Construction d'un gène chimère avec une séquence codant pour l'EPSPS double mutant:

### pRPA-RD-1010:

Création d'une cassette d'expression OTP-EPSPS double mutant pour usage dans les monocotylédones.

Le plasmide pRPA-RD-109 contient le gène de β-glucuronidase(GUS) de *E. coli* contrôlé par le promoteur d'histone H3C4 de maïs (Brignon & coll) associé avec la région non traduite en 5' et le premier intron du gène d'actine de riz *(ActI)* décrit par Mc Elroy D. *et al.* (Plant Molecular Biology 15: 257-268, 1990). Un diagramme de ce plasmide est représenté sur la figure 3. Le plasmide pRPA-RD-109 est digéré avec les enzymes de restriction *NcoI* et *EcoRI,* et le grand fragment d'ADN (environ 5 kb) contenant la séquence vecteur, le gène GUS et le site de polyadénylation NOS est purifié. Le plasmide pRPA-RD-2010 est digéré avec les enzymes de restriction *NcoI* et *EcoRI,* et le fragment d'ADN (environ 1.6 kb) contenant le promoteur H3C4 associé avec la région non traduite en 5' et le premier intron du gène d'actine de riz (*ActI*) est purifié. Les deux fragments d'ADN purifiés sont liés pour créer une cassette d'expression OTP-EPSPS double mutant comprenant le promoteur d'histone H3C4 de maïs (Brignon & coll) associé avec la région non traduite en 5' et le premier intron du gène d'actine de riz *(ActI)* pour contrôler l'expression de la région codante OTP-EPSPS double mutant incorporant le site de polyadénylation NOS.

### 3. construction d'un gène chimère de tolérance à la phosphinothricine (gène bar):

La phosphinothricine acetyl tranferase (PAT) codée par le gène *bar* est une enzyme qui inactive un herbicide, la phosphinothricine (PPT). La PPT inhibe la synthèse de glutamine et provoqie une accumulation rapide d'ammoniaque dans les cellus conduisant à leur mort (Tachibana *et al.* 1986) .

Le plasmide utilisé pour introduire la tolérance à la phosphinothricine comme agent de sélection est obtenu par insertion du gène chimère contenu dans le plasmide pDM 302 dans le vecteur pSP72 de 2462 pb, commercialisé par Promega Corp. (Genbank/ DDBJ database accession number X65332) et contenant le gène de résistance à l'amplicilline.

Le plasmide pDM 302 de 4700pb a été décrit par Cao, J., *et al.* Plant Cell Report 11: 586-591 (1992).

Les différents éléments du gène chimère contenu dans ce plasmide sont:
- le promoteur du gène actine de riz décrit par Mc Elroy D. *et al.* Plant Molecular Biology 15: 257-268 (1990) constitué de 840 pb;
- le premier exon du gène actine de riz constitué de 80 pb;
- le premier intron du gène actine de riz constitué de 450 pb;
- la région codante du gène *bar* de 600 pb excisée du plasmide pIJ41404 décrit par White J*. et al.* Nuc. Acids res. 18: 1862 (1990);
- le terminateur du gène de la nopaline synthase (nos) (zone de polyadénylation du gène *nos* isolé de pTi 37, 250 pb (Bevan M. et al. Nucleics Acids Res. 11: 369-385).

### 4. transformation des cellules de maïs :

La technique du bombardement de particules est utilisée pour introduire la construction génétique. Les plasmides sont purifiés sur colonne Qiagen et coprécipités sur particules de tungstène M10 selon le procédé Klein (Nature 327: 70-73, 1987).

Un mélange de particules métalliques, du plasmide pRPA-RD-1002 et du plasmide de l'exemple 3 décrits ci-dessus, est ensuite bombardé sur des cellules embryogènes de maïs selon le protocole décrit par Gordon-Kamm, W.J. et al. (*Transformation of Maize Cells and Regeneration of Fertile Transgenic Plants,* The Plant Cell, vol. 2, 603-618, July 1990).

### 5. Régénération et utilisation du gène bar comme agent de sélection:

Les cals bombardés sont sélectionnés sur glufosinate jusqu'à l'apparition de secteurs verts. Les cals positifs résistants au glufosinate sont alors convertis en embryons somatiques, puis mis dans les conditions favorisant la germination selon les conditions opératoires décrites par Gordon-Kamm, W.J. et al. (*Transformation of Maize Cells and Regeneration of Fertile Transgenic Plants,* The Plant Cell, vol. 2, 603-618, July 1990). Les jeunes plantes son transférées en serre pour la production de graines.

### 6. Analyse de la descendance des plantes transformées:

Les plantes transformées obtenues ci-dessus sont supposés en partie transgéniques, comprenant un gène hétérologue codant pour OTP/HPPD conférant une tolérance aux isoxazoles comme l'isoxafutole. Ces plantes transformées ont émis du pollen, qui a fécondé des ovules d'un maïs sauvage non transgénique. Les graines obtenues sont sélectionnées sur sable après traitement à l'isoxaflutole.
Le protocole de sélection est le suivant:

800ml de sable de Fontainebleau sont placés dans une barquette de 15 x 20 cm de côtés. Ces barquettes sont alors arrosées par de l'eau et maintenant hydratées par apport d'une solution nutritive constituée de 5ml de Quinoligo® (La Quinoléine) par litre d'eau. Vingt graines de maïs sont placées sur les barquettes, qui sont alors traitées à l'isoxaflutole par pulvérisation à raison de 100g de matière active par hectare (300 µg de matière active par barquette). Les barquettes sont ensuite placées en culture en serre. La phytotoxicité est déterminée 14 jours après la plantation. Selon les conditions ci-dessus, les plantes non transformées présentent 100 % de phytotoxicité alors que les plantes transformées ne présentent pas de phytotoxicité.

Une étude comparative a été réalisée avec 20 lignées de maïs transformé selon l'invention et 20 lignées de maïs transformé avec un gène correspondant pour lequel la séquence codant pour le premier intron de l'actine de riz a été remplacée par la séquence codant pour l'intron adh I de maïs. Après traitement par pulvérisation de très fortes doses d'isoxafutole à raison de 200 g de matière active par hectare (600 µg de matière active par barquette), on obtient les résultats suivants :

| | |
|---|---|
| Intron actine de riz selon l'invention : | 8/20 lignées sont tolérantes |
| Intron adh I de maïs : | 3/20 lignées sont tolérantes |

Les résultats ci-dessus démontrent que l'association du promoteur H3C4 de maïs avec le premier intron de l'actine de riz selon l'invention améliore de manière substantielle l'expression d'une protéine d'intérêt dans les plantes monocotylédones transformées par rapport à l'association du même promoteur H3C4 de maïs avec un autre intron de l'état de la technique.

### LISTE DE SEQUENCES

(iii) NOMBRE DE SEQUENCES: 5
(2) INFORMATIONS POUR LA SEQ ID NO: 1:
   (i) CARACTERISTIQUES DE LA SEQUENCE:
      (A) LONGUEUR: 1021 paires de bases
      (B) TYPE: nucléotide
      (C) NOMBRE DE BRINS: simple
      (D) CONFIGURATION: linéaire
   (ii) TYPE DE MOLECULE: ADN
   (ix) CARACTERISTIQUE:
      (A) NOM/CLE: promoteur H3C4 de mais
      (B) EMPLACEMENT:1..1021
   (xi) DESCRIPTION DE LA SEQUENCE: SEQ ID NO: 1:
   CTTATGTGCA CCATTTACTG TAATGCATAA TCATTTAATT GAATAGCAAA CTTTTCTATT      60
   ACTTCTTTAC TAACATAATT CTTGGTTTTA AAATTCAGTC CTCAACATTC ATTGCTCAAG     120
   TATAAGTTGA GACTGTCAAA ATTTACTATT TTATTTCTTC ATATTTTTTT TCCTTATACA     180
   CATTTTGGGC CTTACAATCC ATCATCTATA TCCATCCTTT CCGGTGTCCT CTAAAAGATT     240
   CCATCCTCTG AATCTTATTC CTCTCCAATA ACGTTCTCTA AATCAGGTCT CTATAAGCAA     300
   TACCTATATT AGAGACATTT TTTATTTTTT GTACATACAT ATTTGTCATA CTCTCAAATG     360
   CATTATACAT ATTTAGTTTT ACTAAACCGA TTATTTAAAG TATTCAAACG GATGAAGAAC     420
   TGTTTAGATA AATTCTATAT ATAGAGAATC CAGTAGCGTT CTCTAAATTT AGATGATTAT     480
   TTAGAGGACG CTGTTAGAAA ACGTAAAAAA TTCTTTGATT ATTTATATTT AGGGTAGAGT     540
   AGCCTTTATG CTTTATAGAT CTTTGGTGGA CCCAGCCTTA TACCGGTTAT TTTCGCGATT     600
   GCGCCTCTCA TTTTCACTCC AGCGCCCCAC ATTTTCACGT TTTCACCGAA GCGCCCAGCC     660
   TGCCTAACCA ACAAATTGGT ACGGTGGCGC GGTTTTCAAA AGAAGTCGGA AACCATCTGC     720
   ACCCACCGAC TAGTAGGCCC TCGGATCCTC CCTGATTAAG TCCTAGCCAA TAGGAGCCCA     780
   GAACCACCCA TCACGCGGAT CGTCCCTACG CTTCCACCTC ATCGGCGCCG TCCATCTCCA     840
   TCCAACACCT ATTCCGTTAC CTTGCCCATC CTCCGAAAAA ATTCTCGGCT CGCGCTCCGC     900
   ACCTACTACA AATACCCATC CCATCACGAC GCATCGCATC ACTGCCAAAT CCCCCAGAAA     960
   ATCAACACCT CCCAATTCCA CGCTGCCACC AACTCGCCGT CCTCCGCGCC AAGCACCAAA    1020
   G                                                                    1021
(2) INFORMATIONS POUR LA SEQ ID NO: 2:
   (i) CARACTERISTIQUES DE LA SEQUENCE:
      (A) LONGUEUR: 454 paires de bases
      (B) TYPE: nucléotide
      (C) NOMBRE DE BRINS: simple
      (D) CONFIGURATION: linéaire
   (ii) TYPE DE MOLECULE: ADN
   (ix) CARACTERISTIQUE:
      (A) NOM/CLE: intron 1 de l'actine de riz
      (B) EMPLACEMENT:1..454
   (xi) DESCRIPTION DE LA SEQUENCE: SEQ ID NO: 2:
   GTAACCACCC CGCCCCTCTC CTCTTTCTTT CTCCGTTTTT TTTTTCGTCT CGGTCTCGAT     60
   CTTTGGCCTT GGTAGTTTGG GTGGGCGAGA GCGGCTTCGT CGCCCAGATC GGTGCGCGGG    120
   AGGGGCGGGA TCTCGCGGCT GGCGTCTCCG GGCGTGAGTC GGCCCGGATC CTCGCGGGGA    180
   ATGGGGCTCT CGGATGTAGA TCTGATCCGC CGTTGTTGGG GGAGATGATG GGGCGTTTAA    240
   AATTTCGCCA TGCTAAACAA GATCAGGAAG AGGGGAAAAG GGCACTATGG TTTATATTTT    300
   TATATATTTC TGCTGCTGCT CGTCAGGCTT AGATGTGCTA GATCTTTCTT TCTTCTTTTT    360
   GTGGGTAGAA TTTGAATCCC TCAGCATTGT TCATCGGTAG TTTTTCTTTT CATGATTTGT    420
   GACAAATGCA GCCTCGTGCG GAGCTTTTTT GTAG                                454
(2) INFORMATIONS POUR LA SEQ ID NO: 3:
   (i) CARACTERISTIQUES DE LA SEQUENCE:
      (A) LONGUEUR: 1565 paires de bases
      (B) TYPE: nucléotide
      (C) NOMBRE DE BRINS: simple
      (D) CONFIGURATION: linéaire
   (ii) TYPE DE MOLECULE: ADN (génomique)
   (ix) CARACTERISTIQUE:
      (A) NOM/CLE: promoteur H3C4 du mais
      (B) EMPLACEMENT:27..1047
   (ix) CARACTERISTIQUE:
      (A) NOM/CLE: intron 1 de l'actine de riz
      (B) EMPLACEMENT:1102..1555
   (ix) CARACTERISTIQUE:
      (A) NOM/CLE: site de clonage synthétique
      (B) EMPLACEMENT : 1..26
   (ix) CARACTERISTIQUE:
      (A) NOM/CLE: séquence de liaison synthétique
      (B) EMPLACEMENT:1048..1069
   (xi) DESCRIPTION DE LA SEQUENCE: SEQ ID NO: 3:
   GAATTCCTGC AGGTCGACGG ATCCCCCTTA TGTGCACCAT TTACTGTAAT GCATAATCAT      60
   TTAATTGAAT AGCAAACTTT TCTATTACTT CTTTACTAAC ATAATTCTTG GTTTTAAAAT     120
   TCAGTCCTCA ACATTCATTG CTCAAGTATA AGTTGAGACT GTCAAAATTT ACTATTTTAT     180
   TTCTTCATAT TTTTTTTCCT TATACACATT TTGGGCCTTA CAATCCATCA TCTATATCCA     240
   TCCTTTCCGG TGTCCTCTAA AAGATTCCAT CCTCTGAATC TTATTCCTCT CCAATAACGT     300
   TCTCTAAATC AGGTCTCTAT AAGCAATACC TATATTAGAG ACATTTTTTA TTTTTTGTAC     360
   ATACATATTT GTCATACTCT CAAATGCATT ATACATATTT AGTTTTACTA AACCGATTAT     420
   TTAAAGTATT CAAACGGATG AAGAACTGTT TAGATAAATT CTATATATAG AGAATCCAGT     480
   AGCGTTCTCT AAATTTAGAT GATTATTTAG AGGACGCTGT TAGAAAACGT AAAAAATTCT     540
   TTGATTATTT ATATTTAGGG TAGAGTAGCC TTTATGCTTT ATAGATCTTT GGTGGACCCA     600
   GCCTTATACC GGTTATTTTC GCGATTGCGC CTCTCATTTT CACTCCAGCG CCCCACATTT     660
   TCACGTTTTC ACCGAAGCGC CCAGCCTGCC TAACCAACAA ATTGGTACGG TGGCGCGGTT     720
   TTCAAAAGAA GTCGGAAACC ATCTGCACCC ACCGACTAGT AGGCCCTCGG ATCCTCCCTG     780
   ATTAAGTCCT AGCCAATAGG AGCCCAGAAC CACCCATCAC GCGGATCGTC CCTACGCTTC     840
   CACCTCATCG GCGCCGTCCA TCTCCATCCA ACACCTATTC CGTTACCTTG CCCATCCTCC     900
   GAAAAAATTC TCGGCTCGCG CTCCGCACCT ACTACAAATA CCCATCCCAT CACGACGCAT     960
   CGCATCACTG CCAAATCCCC CAGAAAATCA ACACCTCCCA ATTCCACGCT GCCACCAACT    1020
   CGCCGTCCTC CGCGCCAAGC ACCAAAGGAA TTGGCCGCCA CCGCGGTGGA GCTCCTCCCC    1080
   CCTCCCCCTC CGCCGCCGCC GGTAACCACC CCGCCCCTCT CCTCTTTCTT TCTCCGTTTT    1140
   TTTTTTCGTC TCGGTCTCGA TCTTTGGCCT TGGTAGTTTG GGTGGGCGAG AGCGGCTTCG    1200
   TCGCCCAGAT CGGTGCGCGG GAGGGGCGGG ATCTCGCGGC TGGCGTCTCC GGGCGTGAGT    1260
   CGGCCCGGAT CCTCGCGGGG AATGGGGCTC TCGGATGTAG ATCTGATCCG CCGTTGTTGG    1320
   GGGAGATGAT GGGGCGTTTA AAATTTCGCC ATGCTAAACA AGATCAGGAA GAGGGGAAAA    1380
   GGGCACTATG GTTTATATTT TTATATATTT CTGCTGCTGC TCGTCAGGCT TAGATGTGCT    1440
   AGATCTTTCT TTCTTCTTTT TGTGGGTAGA ATTTGAATCC CTCAGCATTG TTCATCGGTA    1500
   GTTTTTCTTT TCATGATTTG TGACAAATGC AGCCTCGTGC GGAGCTTTTT TGTAGGTAGA    1560
   CC ATG                                                               1565
(2) INFORMATIONS POUR LA SEQ ID NO: 4:
   (i) CARACTERISTIQUES DE LA SEQUENCE:
      (A) LONGUEUR: 85 paires de bases
      (B) TYPE: oligonucléotide synthétique
      (C) NOMBRE DE BRINS: simple
      (D) CONFIGURATION: linéaire
   (ii) TYPE DE MOLECULE: ADN
   (xi) DESCRIPTION DE LA SEQUENCE: SEQ ID NO: 4:
   AGGGCCCCCT AGGGTTTAAA CGGCCAGTCA GGCCGAATTC GAGCTCGGTA CCCGGGGATC    60
   CTCTAGAGTC GACCTGCAGG CATGC                                          85
(2) INFORMATIONS POUR LA SEQ ID NO: 5:
   (i) CARACTERISTIQUES DE LA SEQUENCE:
      (A) LONGUEUR: 66 paires de bases
      (B) TYPE: oligonucléotide synthétique
      (C) NOMBRE DE BRINS: simple
      (D) CONFIGURATION: linéaire
   (ii) TYPE DE MOLECULE: ADN
   (xi) DESCRIPTION DE LA SEQUENCE: SEQ ID NO: 5:
   CCCTGAACCA GGCTCGAGGG CGCGCCTTAA TTAAAAGCTT GCATGCCTGC AGGTCGACTC    60
   TAGAGG                                                               66

## Revendications

1. Séquence d'ADN, élément de régulation en 5' permettant l'expression d'un gène hétérologue dans une cellule végétale de plante monocotylédone, **caractérisée en ce qu'**elle comprend dans le sens de la transcription une première séquence d'ADN, fragment fonctionnel de la séquence du promoteur H3C4 de maïs, et une deuxième séquence d'ADN, fragment fonctionnel de la séquence du premier intron de l'actine de riz.

2. Séquence selon la revendication 1, **caractérisé en ce que** la séquence du promoteur H3C4 de maïs est le fragment *AluI* du promoteur H3C4 de maïs.

3. Séquence selon la revendication 1, **caractérisée en ce que** le fragment fonctionnel de la séquence du promoteur H3C4 de maïs comprend la séquence d'ADN décrite par l'identificateur de séquence n° 1 (SEQ ID NO:1) ou une séquence homologue présentant un degré d'homologie d'au moins 70% par rapport à ladite séquence.

4. Séquence selon la revendication 3, **caractérisée en ce que** le fragment fonctionnel de la séquence du promoteur H3C4 de maïs consiste en la séquence d'ADN décrite par l'identificateur de séquence n° 1.

5. Séquence selon l'une des revendications 1 à 4, **caractérisée en ce que** le fragment fonctionnel du premier intron de l'actine de riz comprend la séquence d'ADN décrite par l'identificateur de séquence n° 2 (SEQ ID NO:2) ou une séquence homologue présentant un degré d'homologie d'au moins 70% par rapport à ladite séquence.

6. Séquence selon la revendication 5, **caractérisée en ce que** le fragment fonctionnel du premier intron de l'actine de riz consiste en la séquence d'ADN décrite par l'identificateur de séquence n° 2.

7. Séquence d'ADN élément de régulation en 5' permettant l'expression d'un gène hétérologue dans une cellule végétale de plante monocotylédone, **caractérisée en ce qu'**elle comprend la séquence d'ADN représentée par l'identificateur de séquence n° 3 (SEQ ID NO:3) ou une séquence homologue présentant un degré d'homologie d'au moins 70% par rapport à ladite séquence.

8. Séquence d'ADN selon la revendication 7, **caractérisée en ce qu'**elle consiste en la séquence d'ADN représentée par l'identificateur de séquence n° 3.

9. Gène chimère comprenant une séquence codante ainsi que des éléments de régulation en position 5' et 3' hétérologues pouvant fonctionner dans les cellules végétales de plantes monocotylédones ou les plantes monocotylédones, **caractérisé en ce que** les éléments de régulation en 5' comprennent la séquence d'ADN selon l'une des revendications 1 à 8.

10. Gène chimère selon la revendication 9, **caractérisé en ce que** la séquence codante est choisi parmi un gène codant pour un marqueur de sélection, un gène conférant à la plante monocotylédone transformée de nouvelles propriétés agronomiques, et un gène d'amélioration de la qualité agronomique de la plante monocotylédone transformée.

11. Gène chimère selon la revendication 10, **caractérisé en ce que** le gène conférant de nouvelles propriétés agronomiques aux plantes monocotylédones transformées, est choisi parmi un gène conférant une tolérance à certains herbicides, un gène conférant une tolérance à certains insectes et un gène conférant une tolérance à certaines maladies.

12. Gène chimère selon la revendication 11, **caractérisé en ce que** le gène conférant une tolérance à certains herbicides, est choisi parmi le gène Bar conférant une tolérance au bialaphos, un gène codant pour une enzyme 5-enolpyruvylshikimate-3-phosphate synthase (EPSPS) appropriée conférant une résistance aux herbicides ayant l'EPSPS comme cible, le gène codant pour la glyphosate oxydoréductase et un gène codant pour une hydroxy-phényl pyruvate dioxygénase (HPPD) conférant une tolérance aux herbicides ayant pour cible l'HPPD.

13. Gène chimère selon la revendication 12, **caractérisé en ce que** le gène conférant une tolérance à certains herbicides est choisi parmi un gène codant pour une EPSPS et un gène codant pour une HPPD.

14. Gène chimère selon la revendication 13, **caractérisé en ce que** le gène codant pour une EPSPS est choisi parmi l'EPSPS double mutant et le CP4.

15. Gène chimère selon l'une des revendications 13 ou 14, **caractérisé en ce que** la séquence codant pour une EPSPS ou une HPPD est précédée par une séquence codant pour un peptide de transit.

16. Gène chimère selon la revendication 15, **caractérisé en ce que** la séquence codant pour un peptide de transit est une séquence codant pour un peptide de transit double comprenant, dans le sens de la transcription, une séquence codant pour un peptide de transit d'un gène végétal codant pour une enzyme à localisation plastidiale, une partie de séquence de la partie mature N-terminale d'un gène végétal codant pour une enzyme à localisation plastidiale, puis une séquence codant pour un second peptide de transit d'un gène végétal codant pour une enzyme à localisation plastidiale.

17. Gène chimère **caractérisé en ce qu'**il comprend dans le sens de la transcription, une séquence de régulation en 5' définie selon l'une des revendications 1 à 8, liée de manière fonctionnelle à une séquence codant pour une protéine de fusion peptide de transit/protéine d'intérêt, liée de manière fonctionnelle à une séquence de régulation en 3'.

18. Gène chimère selon la revendication 17, **caractérisé en ce que** la protéine d'intérêt est une enzyme conférant une tolérance à certains herbicide selon l'une des revendications 12 à 14.

19. Gène chimère selon la revendication 18, **caractérisé en ce que** la séquence codant pour une protéine de fusion peptide de transit/protéine d'intérêt est choisie parmi la séquence codant pour la protéine de fusion OTP/EPSPS double mutant et la séquence codant pour la protéine de fusion OTP/CP4.

20. Vecteur de clonage ou d'expression pour la transformation d'une cellule végétale ou d'une plante, **caractérisé en ce qu'**il comprend outre le gène chimère selon l'une des revendications 9 à 19, au moins une origine de réplication.

21. Vecteur selon la revendication 20, **caractérisé en ce qu'**il est un plasmide.

22. Procédé de transformation des cellules végétales, **caractérisé en ce que** l'on intègre un gène chimère selon l'une des revendications 9 à 19.

23. Cellule végétale, **caractérisée en ce qu'**elle contient au moins un gène chimère selon l'une des revendications 9 à 19.

24. Plante transformée, **caractérisée en ce qu'**elle est régénérée à partir des cellules selon la revendication 23.

25. Plante transformée, **caractérisée en ce qu'**elle est issue de la culture et/ou du croisement d'une plante transformée selon la revendication 24.

26. Graine de plante transformée selon l'une des revendications 24 ou 25.

27. Procédé de contrôle des mauvaises herbes dans une surface d'un champ comprenant des graines ou des plantes transformées avec un gène chimère comprenant une séquence codant pour une enzyme conférant une tolérance à un herbicide particulier, lequel procédé consiste à appliquer dans la dite surface du champ une dose dudit herbicide particulier toxique pour les dites mauvaises herbes, sans toutefois affecter de manière substantielle les graines ou plantes transformée avec le dit gène chimère, **caractérisé en ce que** le dit gène chimère est défini selon l'une des revendications 12 à 19.

28. Procédé de culture des plantes transformées avec un gène chimère comprenant une séquence codant pour une enzyme conférant une tolérance à un herbicide particulier, lequel procédé consiste à planter les graines des dites plantes transformées dans une surface d'un champ approprié pour la culture des dites plantes, à appliquer sur la dite surface du dit champ une dose toxique pour les mauvaises herbes du dit herbicide particulier en cas de présence de mauvaises herbes, sans affecter de manière substantielle les dites graines ou les dites plantes transformées, puis à récolter les plantes cultivées lorsqu'elles arrivent à la maturité souhaitée et éventuellement à séparer les graines des plantes récoltées, **caractérisé en ce** ledit gène chimère est un gène chimère selon l'une des revendications 12 à 19.

29. Procédé selon la revendication 28, **caractérisé en ce qu'**on applique l'herbicide particulier en présemis, en prélevée ou en postlevée de la culture.

## Patentansprüche

1. DNA-Sequenz, bei der es sich um ein 5'-Regulationselement, das die Expression eines heterologen Gens in einer Pflanzenzelle von monokotyledonen Pflanzen ermöglicht, handelt, **dadurch gekennzeichnet, daß** sie in Transkriptionsrichtung eine erste DNA-Sequenz, bei der es sich um ein funktionfähiges Fragment der H3C4-Promotorsequenz aus Mais handelt, sowie eine zweite DNA-Sequenz, bei der es sich um ein funktionsfähiges Fragment der Sequenz des ersten Actin-Introns aus Reis handelt, umfaßt.

2. Sequenz nach Anspruch 1, **dadurch gekennzeichnet, daß** es sich bei der Sequenz des H3C4-Promotors aus Mais um das AluI-Fragment des H3C4-Promotors aus Mais handelt.

3. Sequenz nach Anspruch 1, **dadurch gekennzeichnet, daß** das funktionsfähige Fragment der Sequenz des . H3C4-Promotors aus Mais die durch die Sequenzbeschreibung Nr. 1 (SEQ ID Nr. 1) beschriebene DNA-Sequenz oder eine homologe Sequenz mit einem Homologiegrad von mindestens 70% in bezug auf diese Sequenz umfaßt.

4. Sequenz nach Anspruch 3, **dadurch gekennzeichnet, daß** das funktionsfähige Fragment der Sequenz des H3C4-Promotors aus Mais aus der durch die Sequenzbeschreibung Nr. 1 beschriebenen DNA-Sequenz besteht.

5. Sequenz nach einem der Ansprüche 1 bis 4, **dadurch gekennzeichnet, daß** das funktionsfähige Fragment der Sequenz des ersten Actin-Introns aus Reis die durch die Sequenzbeschreibung Nr. 2 (SEQ ID Nr. 2) beschriebene DNA-Sequenz oder eine homologe Sequenz mit einem Homologiegrad von mindestens 70% in bezug auf diese Sequenz umfaßt.

6. Sequenz nach Anspruch 5, **dadurch gekennzeichnet, daß** das funktionsfähige Fragment der Sequenz des ersten Actin-Introns aus Reis aus der durch die Sequenzbeschreibung Nr. 2 beschriebenen DNA-Sequenz besteht.

7. DNA-Sequenz, bei der es sich um ein 5'-Regulationselement, das die Expression eines heterologen Gens in einer Pflanzenzelle von monokotyledonen Pflanzen ermöglicht, handelt, **dadurch gekennzeichnet, daß** sie die durch die Sequenzbeschreibung Nr. 3 (SEQ ID Nr. 3) beschriebene DNA-Sequenz oder eine homologe Sequenz mit einem Homologiegrad von mindestens 70% in bezug auf diese Sequenz aufweist, umfaßt.

8. Sequenz nach Anspruch 7, **dadurch gekennzeichnet, daß** sie aus der durch die Sequenzbeschreibung Nr. 3 beschriebenen DNA-Sequenz besteht.

9. Chimäres Gen, das eine Codiersequenz sowie heterologe 5'- und 3'-Regulationselemente, die in Monokotyledonen-Pflanzenzellen oder in monokotyledonen Pflanzen funktionsfähig sind, umfaßt, **dadurch gekennzeichnet, daß** die 5'-Regulationselemente die DNA-Sequenz nach einem der Ansprüche 1 bis 8 umfassen.

10. Chimäres Gen nach Anspruch 9, **dadurch gekennzeichnet, daß** die Codiersequenz aus der Gruppe Gen, das für einen Selektionsmarker codiert, Gen, das der transformierten monokotyledonen Pflanze neue agronomische Eigenschaften verleiht und Gen zur Verbesserung der agronomischen Qualität der transformierten monokotyledonen Pflanze stammt.

11. Chimäres Gen nach Anspruch 10, **dadurch gekennzeichnet, daß** das Gen, das den transformierten monokotyledonen Pflanzen neue agronomische Eigenschaften verleiht, aus der Gruppe Gen, das eine Toleranz gegenüber gewissen Herbiziden verleiht, Gen, das eine Toleranz gegenüber gewissen Insekten verleiht und Gen, das eine Toleranz gegenüber gewissen Krankheiten verleiht, stammt.

12. Chimäres Gen nach Anspruch 11, **dadurch gekennzeichnet, daß** das Gen, das eine Toleranz gegenüber gewissen Herbiziden verleiht, aus der Gruppe Bar-Gen, das eine Toleranz gegenüber Bialaphos verleiht, Gen, das für ein geeignetes 5-Enolpyruvylshikimat-3-phosphatsynthase (EPSPS) -Enzym, das eine Resistenz gegenüber Herbiziden, denen die EPSPS als Angriffspunkt dient, vermittelt, das Gen, das für die Glyphosat-Oxidoreduktase codiert und Gen, das für eine Hydroxyphenylpyruvatdioxygenase (HPPD), die eine Toleranz gegenüber Herbiziden, denen die HPPD als Angriffspunkt dient, vermittelt, codiert, stammt.

13. Chimäres Gen nach Anspruch 12, **dadurch gekennzeichnet, daß** das Gen, das eine Toleranz gegenüber gewissen Herbiziden verleiht, aus der Gruppe Gen, das für eine EPSPS codiert und Gen, das für eine HPPD codiert, stammt.

14. Chimäres Gen nach Anspruch 13, **dadurch gekennzeichnet, daß** das Gen, das für eine EPSPS codiert, aus der Reihe EPSPS-Doppelmutante und CP4 stammt.

15. Chimäres Gen nach Anspruch 13 oder 14, **dadurch gekennzeichnet, daß** der Sequenz, die für eine EPSPS oder eine HPPD codiert, eine Sequenz, die für ein Leitpeptid codiert, vorangestellt ist.

16. Chimäres Gen nach Anspruch 15, **dadurch gekennzeichnet, daß** es sich bei der Sequenz, die für ein Leitpeptid codiert, um eine Sequenz handelt, die für ein doppeltes Leitpeptid codiert und die in Transkriptionsrichtung eine Sequenz, die für ein Leitpeptid eines pflanzlichen Gens, das für ein Enzym für plastidäre Lokalisierung codiert, codiert, einen Sequenzabschnitt des reifen N-terminalen Abschnitts eines pflanzlichen Gens, das für ein Enzym für plastidäre Lokalisierung codiert, anschließend eine Sequenz, die für ein zweites Transidpeptid eines pflanzlichen Gens, das für ein Enzym für plastidäre Lokalisierung codiert, codiert, umfaßt.

17. Chimäres Gen, **dadurch gekennzeichnet, daß** es in Transkriptionsrichtung eine 5'-Regulationssequenz nach einem der Ansprüche 1 bis 8 in operativer Verknüpfung mit einer Sequenz, die für ein Fusionsprotein Leitpeptid/interessierendes Protein codiert und die operativ mit einer 3'-Regulationssequenz verknüpft ist, umfaßt.

18. Chimäres Gen nach Anspruch 17, **dadurch gekennzeichnet, daß** es sich bei dem interessierenden Protein um ein Enzym nach einem der Ansprüche 12 bis 14, das eine Toleranz gegenüber gewissen Herbiziden vermittelt, handelt.

19. Chimäres Gen nach Anspruch 18, **dadurch gekennzeichnet, daß** die für ein Fusionsprotein Leitpeptid/interessierendes Protein codierende Sequenz aus der Reihe Sequenz, die für das Fusionsprotein OTP/EPSPS-Doppelmutante codiert und Sequenz, die für das Fusionsprotein OTP/CP4 codiert, stammt.

20. Klonierungs- oder Expressionsvektor für die Transformation einer Pflanzenzelle oder einer Pflanze, **dadurch gekennzeichnet, daß** er außer dem chimären Gen nach einem der Ansprüche 9-19 mindestens einen Replikationsursprung umfaßt.

21. Vektor nach Anspruch 20, **dadurch gekennzeichnet, daß** es sich um ein Plasmid handelt.

22. Verfahren zur Transformation von Pflanzenzellen, **dadurch gekennzeichnet, daß** man ein chimäres Gen nach einem der Ansprüche 9-19 integriert.

23. Pflanzenzelle, **dadurch gekennzeichnet, daß** sie mindestens ein chimäres Gen nach einem der Ansprüche 9-19 enthält.

24. Transformierte Pflanze, **dadurch gekennzeichnet, daß** sie aus Zellen nach Anspruch 23 regeneriert wird.

25. Transformierte Pflanze, **dadurch gekennzeichnet, daß** sie aus der Kultur und/oder einer Kreuzung einer transformierten Pflanze nach Anspruch 24 entstanden ist.

26. Samen einer transformierten Pflanze nach Anspruch 24 oder 25.

27. Verfahren zur Bekämpfung von Unkräutern in der Oberfläche eines Feldes enthaltend Samen oder Pflanzen, die mit einem chimären Gen umfassend eine Sequenz, die für ein Enzym, das eine Toleranz gegenüber ein bestimmtes Herbizid vermittelt, codiert, transformiert worden sind, wobei dieses Verfahren darin besteht, daß man in diese Oberfläche des Feldes eine Dosis dieses bestimmten Herbizids, die für diese Unkräuter toxisch ist, ohne jedoch die mit dem chimären Gen transformierten Samen oder Pflanzen wesentlich zu schädigen, ausbringt, **dadurch gekennzeichnet, daß** das chimäre Gen nach einem der Ansprüche 12-19 definiert ist.

28. Verfahren zur Kultivierung von mit einem chimären Gen umfassend eine Sequenz, die für ein Enzym, das eine Toleranz gegenüber einem bestimmten Herbizid vermittelt, codiert, transformierten Pflanzen, wobei das Verfahren darin besteht, daß man die Samen dieser transformierten Pflanzen in eine Oberfläche des Felds, das sich für die Kultur dieser Pflanzen eignet, pflanzt, bei Vorhandensein von Unkräutern auf die Oberfläche des Feldes eine für die Unkräuter toxische Dosis dieses bestimmten Herbizids ausbringt, ohne daß die transformierten Samen oder Pflanzen wesentlich geschädigt werden, anschließend die kultivierten Pflanzen, nachdem diese den gewünschten Reifegrad erlangt haben, erntet sowie gegebenenfalls die Samen der geernteten Pflanzen abtrennt, **dadurch gekennzeichnet, daß** es sich bei dem chimären Gen um ein chimäres Gen nach einem der Ansprüche 12-19 handelt.

29. Verfahren nach Anspruch 28, **dadurch gekennzeichnet, daß** man das bestimmte Herbizid vor dem Anbau, vor dem Auflaufen oder nach dem Auflaufen der Kultur ausbringt.

## Claims

1. DNA sequence, a 5' regulatory element allowing the expression of a heterologous gene in a plant cell from a monocotyledonous plant, **characterized in that** it comprises, in the direction of transcription, a first DNA sequence, which is a functional fragment of the sequence of the maize H3C4 promoter, and a second DNA sequence, which is a functional fragment of the sequence of the first intron of rice actin.

2. Sequence according to Claim 1, **characterized in that** the sequence of the maize H3C4 promoter is the *AluI* fragment of the maize H3C4 promoter.

3. Sequence according to Claim 1, **characterized in that** the functional fragment of the sequence of the maize H3C4 promoter comprises the DNA sequence described by the sequence identifier No. 1 (SEQ ID NO: 1) or a homologous sequence having a degree of homology of at least 70% relative to the said sequence.

4. Sequence according to Claim 3, **characterized in that** the functional fragment of the sequence of the maize H3C4 promoter consists of the DNA sequence described by the sequence identifier No. 1.

5. Sequence according to one of Claims 1 to 4, **characterized in that** the functional fragment of the first intron of rice actin comprises the DNA sequence described by the sequence identifier No. 2 (SEQ ID NO: 2) or a homologous sequence having a degree of homology of at least 70% relative to the said sequence.

6. Sequence according to Claim 5, **characterized in that** the functional fragment of the first intron of rice actin consists of the DNA sequence described by the sequence identifier No. 2.

7. DNA sequence, a 5' regulatory element allowing the expression of a heterologous gene in a plant cell from a monocotyledonous plant, **characterized in that** it comprises the DNA sequence represented by the sequence identifier No. 3 (SEQ ID NO: 3) or a homologous sequence having a degree of homology of at least 70% relative to the said sequence.

8. DNA sequence according to Claim 7, **characterized in that** it consists of the DNA sequence represented by the sequence identifier No. 3.

9. Chimeric gene comprising a coding sequence as well as heterologous regulatory elements at the 5' and 3' positions capable of functioning in plant cells from monocotyledonous plants or in monocotyledonous plants, **characterized in that** the 5' regulatory elements comprise the DNA sequence according to one of Claims 1 to 8.

10. Chimeric gene according to Claim 9, **characterized in that** the coding sequence is chosen from a gene encoding a selectable marker, a gene conferring on the transformed monocotyledonous plant new agronomic properties, and a gene for enhancing the agronomic quality of the transformed monocotyledonous plant.

11. Chimeric gene according to Claim 10, **characterized in that** the gene conferring new agronomic properties on the transformed monocotyledonous plants is chosen from a gene conferring tolerance to certain herbicides, a gene conferring tolerance to certain insects and a gene conferring tolerance to certain diseases.

12. Chimeric gene according to Claim 11, **characterized in that** the gene conferring tolerance to certain herbicides is chosen from the Bar gene conferring tolerance to bialaphos, a gene encoding an appropriate 5-enolpyruvylshikimate-3-phosphate synthase (EPSPS) enzyme conferring resistance to herbicides having EPSPS as target, the gene encoding glyphosate oxidoreductase and a gene encoding a hydroxyphenylpyruvate dioxygenase (HPPD) conferring tolerance to herbicides having HPPD as target.

13. Chimeric gene according to Claim 12, **characterized in that** the gene conferring tolerance to certain herbicides is chosen from a gene encoding an EPSPS and a gene encoding an HPPD.

14. Chimeric gene according to Claim 13, **characterized in that** the gene encoding an EPSPS is chosen from the double-mutant EPSPS and CP4.

15. Chimeric gene according to either of Claims 13 and 14, **characterized in that** the sequence encoding an EPSPS or an HPPD is preceded by a sequence encoding a transit peptide.

16. Chimeric gene according to Claim 15, **characterized in that** the sequence encoding a transit peptide is a sequence encoding a double transit peptide comprising, in the direction of transcription, a sequence encoding a transit peptide for a plant gene encoding a plastid localization enzyme, a portion of sequence of the mature N-terminal portion of a plant gene encoding a plastid localization enzyme, and then a sequence encoding a second transit peptide for a plant gene encoding a plastid localization enzyme.

17. Chimeric gene **characterized in that** it comprises, in the direction of transcription, a 5' regulatory sequence defined according to one of Claims 1 to 8, functionally linked to a sequence encoding a fusion protein transit peptide/protein of interest, functionally linked to a 3' regulatory sequence.

18. Chimeric gene according to Claim 17, **characterized in that** the protein of interest is an enzyme conferring tolerance to certain herbicides according to one of Claims 12 to 14.

19. Chimeric gene according to Claim 18, **characterized in that** the sequence encoding a fusion protein transit peptide/protein of interest is chosen from the sequence encoding the fusion protein OTP/double-mutant EPSPS and the sequence encoding the fusion protein OTP/CP4.

20. Cloning or expression vector for the transformation of a plant cell or of a plant, **characterized in that** it comprises, in addition to the chimeric gene according to one of Claims 9 to 19 or 23, at least one replication origin.

21. Vector according to Claim 20, **characterized in that** it is a plasmid.

22. Method of transforming plant cells, **characterized in that** a chimeric gene according to one of Claims 9 to 19 is integrated.

23. Plant cell, **characterized in that** it contains at least one chimeric gene according to one of Claims 9 to 19.

24. Transformed plant, **characterized in that** it is regenerated from the cells according to Claim 23.

25. Transformed plant, **characterized in that** it is derived from the culture and/or the crossing of a transformed plant according to Claim 24.

26. Seed of a transformed plant according to either of Claims 24 and 25.

27. Method of controlling weeds in an area of a field comprising seeds or plants transformed with a chimeric gene comprising a sequence encoding an enzyme conferring tolerance to a particular herbicide, which method consists in applying to the said area of the field a dose of the said particular herbicide which is toxic to the said weeds, without, however, substantially affecting the seeds or plants transformed with the said chimeric gene, **characterized in that** the said chimeric gene is defined according to one of Claims 12 to 19.

28. Method of culturing plants transformed with a chimeric gene comprising a sequence encoding an enzyme conferring tolerance to a particular herbicide, which method consists in planting the seeds of the said transformed plants in an area of a field which is appropriate for the culture of the said plants, in applying to the said area of the said field a dose of the said particular herbicide which is toxic to weeds should weeds be present, without substantially affecting the said seeds or the said transformed plants, and then in harvesting the cultivated plants when they reach the desired maturity and optionally in separating the seeds from the harvested plants, **characterized in that** the said chimeric gene is a chimeric gene according to one of Claims 12 to 19.

29. Method according to Claim 28, **characterized in that** the particular herbicide is applied before sowing, before emergence or after emergence of the crop.
